# EUROPEAN PATENT APPLICATION

(11) **EP 1 867 660 A1**
(43) Date of publication of application: **19.12.2007**
(21) Application number: 06012262.9
(22) Date of filing: 14.06.2006
(51) Int. Cl.: C07K 19/00, C12N 15/62

(54) **Proteolytically cleavable fusion protein comprising a blood coagulation factor**

(71) Applicant: CSL Behring GmbH, 35041 Marburg (DE)
(72) Inventor: Metzner, Hubert, Dr., 35041 Marburg (DE); Weimar, Thomas, Dr., 35075 Gladenbach (DE); Schulte, Stefan, Dr., 35043 Marburg (DE)

(57) **Abstract**

The invention relates to therapeutic fusion proteins in which a coagulation factor is fused to a half-life enhancing polypeptide, and both are connected by a linker peptide that is proteolytically cleavable. The cleavage of such linkers liberates the coagulation factor from any activity-compromising steric hindrance caused by the half-life enhancing polypeptide and, thereby, allows the generation of fusion proteins with high molar specific activity. Furthermore, the fact that the linker is cleavable can enhance the rates of inactivation and/or elimination after cleavage respectively activation compared to the corresponding therapeutic fusion protein without cleavable linker.

SEQUENCE LISTING

<110>  ZLB Behring GmbH

<120>  Proteolytically cleavable fusion proteins with high molar
       specific activity

<130>  2006_M004_A115

<160>  30

<170>  PatentIn version 3.3

<210>  1
<211>  585
<212>  PRT
<213>  Homo sapiens

<400>  1

<210>  2
<211>  415
<212>  PRT
<213>  Homo sapiens

<400>  2

Tyr Asn Ser Gly Lys Leu Glu Glu Phe Val Gln Gly Asn Leu Glu Arg
1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val

115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile
        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys

385                 390                 395                 400

Val Ser Arg Tyr Val Asn Trp Ile Lys Glu Lys Thr Lys Leu Thr
                405                 410                 415

<210>  3
<211>  1386
<212>  DNA
<213>  Homo sapiens

<400>  3
atgcagcgcg tgaacatgat catggcagaa tcaccaggcc tcatcaccat ctgcctttta      60

ggatatctac tcagtgctga atgtacagtt tttcttgatc atgaaaacgc caacaaaatt     120

ctgaatcggc caaagaggta taattcaggt aaattggaag agtttgttca agggaacctt     180

gagagagaat gtatggaaga aaagtgtagt tttgaagaag cacgagaagt ttttgaaaac     240

actgaaagaa caactgaatt ttggaagcag tatgttgatg gagatcagtg tgagtccaat     300

ccatgtttaa atggcggcag ttgcaaggat gacattaatt cctatgaatg ttggtgtccc     360

tttggatttg aaggaaagaa ctgtgaatta gatgtaacat gtaacattaa gaatggcaga     420

tgcgagcagt tttgtaaaaa tagtgctgat aacaaggtgg tttgctcctg tactgaggga     480

tatcgacttg cagaaaacca gaagtcctgt gaaccagcag tgccatttcc atgtggaaga     540

gtttctgttt cacaaacttc taagctcacc cgtgctgaga ctgtttttcc tgatgtggac     600

tatgtaaatt ctactgaagc tgaaaccatt ttggataaca tcactcaaag cacccaatca     660

tttaatgact tcactcgggt tgttggtgga gaagatgcca aaccaggtca attcccttgg     720

caggttgttt tgaatggtaa agttgatgca ttctgtggag gctctatcgt taatgaaaaa     780

tggattgtaa ctgctgccca ctgtgttgaa actggtgtta aaattacagt tgtcgcaggt     840

gaacataata ttgaggagac agaacataca gagcaaaagc gaaatgtgat tcgaattatt     900

cctcaccaca actacaatgc agctattaat aagtacaacc atgacattgc ccttctggaa     960

ctggacgaac ccttagtgct aaacagctac gttacaccta tttgcattgc tgacaaggaa    1020

tacacgaaca tcttcctcaa atttggatct ggctatgtaa gtggctgggg aagagtcttc    1080

cacaaaggga gatcagcttt agttcttcag taccttagag ttccacttgt tgaccgagcc    1140

acatgtcttc gatctacaaa gttcaccatc tataacaaca tgttctgtgc tggcttccat    1200

gaaggaggta gagattcatg tcaaggagat agtgggggac cccatgttac tgaagtggaa    1260

gggaccagtt tcttaactgg aattattagc tggggtgaag agtgtgcaat gaaaggcaaa    1320

tatggaatat ataccaaggt atcccggtat gtcaactgga ttaaggaaaa aacaaagctc    1380

acttaa                                                                1386

<210>  4
<211>  1830
<212>  DNA
<213>  Homo sapiens

<400>  4

atgaagtggg taacctttat ttcccttctt tttctcttta gctcggctta ttccaggggt 60
gtgtttcgtc gagatgcaca caagagtgag gttgctcatc ggtttaaaga tttgggagaa 120
gaaaatttca aagccttggt gttgattgcc tttgctcagt atcttcagca gtgtccattt 180
gaagatcatg taaaattagt gaatgaagta actgaatttg caaaaacatg tgttgctgat 240
gagtcagctg aaaattgtga caaatcactt catacccttt ttggagacaa attatgcaca 300
gttgcaactc ttcgtgaaac ctatggtgaa atggctgact gctgtgcaaa acaagaacct 360
gagagaaatg aatgcttctt gcaacacaaa gatgacaacc caaacctccc ccgattggtg 420
agaccagagg ttgatgtgat gtgcactgct tttcatgaca atgaagagac atttttgaaa 480
aaatacttat atgaaattgc cagaagacat ccttactttt atgccccgga actccttttc 540
tttgctaaaa ggtataaagc tgcttttaca gaatgttgcc aagctgctga taaagctgcc 600
tgcctgttgc caaagctcga tgaacttcgg gatgaaggga aggcttcgtc tgccaaacag 660
agactcaagt gtgccagtct ccaaaaattt ggagaaagag ctttcaaagc atgggcagta 720
gctcgcctga gccagagatt tcccaaagct gagtttgcag aagtttccaa gttagtgaca 780
gatcttacca aagtccacac ggaatgctgc catggagatc tgcttgaatg tgctgatgac 840
agggcggacc ttgccaagta tatctgtgaa aatcaagatt cgatctccag taaactgaag 900
gaatgctgtg aaaaacctct gttggaaaaa tcccactgca ttgccgaagt ggaaaatgat 960
gagatgcctg ctgacttgcc ttcattagct gctgattttg ttgaaagtaa ggatgtttgc 1020
aaaaactatg ctgaggcaaa ggatgtcttc ctgggcatgt ttttgtatga atatgcaaga 1080
aggcatcctg attactctgt cgtgctgctg ctgagacttg ccaagacata tgaaaccact 1140
ctagagaagt gctgtgccgc tgcagatcct catgaatgct atgccaaagt gttcgatgaa 1200
tttaaacctc ttgtggaaga gcctcagaat ttaatcaaac aaaattgtga gctttttgag 1260
cagcttggag agtacaaatt ccagaatgcg ctattagttc gttacaccaa gaaagtaccc 1320
caagtgtcaa ctccaactct tgtagaggtc tcaagaaacc taggaaaagt gggcagcaaa 1380
tgttgtaaac atcctgaagc aaaaagaatg ccctgtgcag aagactatct atccgtggtc 1440
ctgaaccagt tatgtgtgtt gcatgagaaa acgccagtaa gtgacagagt caccaaatgc 1500
tgcacagaat ccttggtgaa caggcgacca tgcttttcag ctctggaagt cgatgaaaca 1560
tacgttccca aagagtttaa tgctgaaaca ttcaccttcc atgcagatat atgcacactt 1620
tctgagaagg agagacaaat caagaaacaa actgcacttg ttgagctcgt gaaacacaag 1680
cccaaggcaa caaaagagca actgaaagct gttatggatg atttcgcagc ttttgtagag 1740
aagtgctgca aggctgacga taaggagacc tgctttgccg aggagggtaa aaaacttgtt 1800
gctgcaagtc aagctgcctt aggcttataa 1830

<210> 5
<211> 21
<212> DNA
<213> Artificial Sequence

<220>
<223>  Artificial

<400>  5
ccactttcac aatctgctag c                                                21


<210>  6
<211>  23
<212>  DNA
<213>  Artificial Sequence

<220>
<223>  Artificial

<400>  6
caattccaat gaattaacct tgg                                              23


<210>  7
<211>  21
<212>  DNA
<213>  Artificial Sequence

<220>
<223>  Artificial

<400>  7
atgcagcgcg tgaacatgat c                                                21


<210>  8
<211>  25
<212>  DNA
<213>  Artificial Sequence

<220>
<223>  Artificial

<400>  8
tcattaagtg agctttgttt tttcc                                            25


<210>  9
<211>  21
<212>  DNA
<213>  Artificial Sequence

<220>
<223>  Artificial

<400>  9
gattcgaatt cgcccttatg c                                                21


<210>  10
<211>  32
<212>  DNA
<213>  Artificial Sequence

<220>
<223>  Artificial

<400>  10
cgctcgaggt gagctttgtt ttttccttaa tc                                    32


<210>  11

<211>  52
<212>  DNA
<213>  Artificial Sequence

<220>
<223>  Artificial

<400>  11
ctcgagcggg ggatctggcg ggtctggagg ctctggaggg tcgggaggct ct          52


<210>  12
<211>  46
<212>  DNA
<213>  Artificial Sequence

<220>
<223>  Artificial

<400>  12
ggatccagat cccccagagc ctccagagcc tcccgaccct ccagag          46


<210>  13
<211>  56
<212>  DNA
<213>  Artificial Sequence

<220>
<223>  Artificial

<400>  13
ctcgagcaat ggatctggcg ggtctggagg ctctggaggg tcgaatggct ctggag          56


<210>  14
<211>  64
<212>  DNA
<213>  Artificial Sequence

<220>
<223>  Artificial

<400>  14
ggatccgttc cctccagacc cgccagatcc cccagagcct ccagagccat tcgaccctcc          60

agag          64


<210>  15
<211>  46
<212>  DNA
<213>  Artificial Sequence

<220>
<223>  Artificial

<400>  15
cctcgagctc tgtgagccag acctccaagc tcaccagggc cgagac          46


<210>  16
<211>  44
<212>  DNA
<213>  Artificial Sequence

<220>
<223>  Artificial

<400>  16
gggatccgtc cacatcaggg aagacagtct cggccctggt gagc                        44


<210>  17
<211>  33
<212>  DNA
<213>  Artificial Sequence

<220>
<223>  Artificial

<400>  17
ggaaaaaaca aagctcactt ctgtgagcca gac                                    33


<210>  18
<211>  33
<212>  DNA
<213>  Artificial Sequence

<220>
<223>  Artificial

<400>  18
gtctggctca cagaagtgag ctttgttttt tcc                                    33


<210>  19
<211>  38
<212>  DNA
<213>  Artificial Sequence

<220>
<223>  Artificial

<400>  19
cctcgagcag agcttcaatg acttcacccg ggtggtgg                               38


<210>  20
<211>  41
<212>  DNA
<213>  Artificial Sequence

<220>
<223>  Artificial

<400>  20
gggatccatc ctccccgccc accacccggg tgaagtcatt g                           41


<210>  21
<211>  34
<212>  DNA
<213>  Artificial Sequence

<220>
<223>  Artificial

<400>  21
ggaaaaaaca aagctcactc agagcttcaa tgac                                   34


<210>  22
<211>  34
<212>  DNA

<213>  Artificial Sequence

<220>
<223>  Artificial

<400>  22
gtcattgaag ctctgagtga gctttgtttt ttcc                                    34


<210>  23
<211>  31
<212>  DNA
<213>  Artificial Sequence

<220>
<223>  Artificial

<400>  23
gtgggatccg atgcacacaa gagtgaggtt g                                       31


<210>  24
<211>  35
<212>  DNA
<213>  Artificial Sequence

<220>
<223>  Artificial

<400>  24
cacggatccc tataagccta aggcagcttg acttg                                   35


<210>  25
<211>  18
<212>  DNA
<213>  Artificial Sequence

<220>
<223>  Artificial

<400>  25
caaggagacg ggcgctcc                                                      18


<210>  26
<211>  19
<212>  DNA
<213>  Artificial Sequence

<220>
<223>  Artificial

<400>  26
   gcccaaggag gggattggc                                                      19


<210>  27
<211>  30
<212>  DNA
<213>  Artificial Sequence

<220>
<223>  Artificial

<400>  27
gtggaattca tggagctgag gccctggttg                                         30

<210>  28
<211>  38
<212>  DNA
<213>  Artificial Sequence

<220>
<223>  Artificial

<400>  28
cacgcggccg ctcactacag ccgttgcccc gcctccac                                38


<210>  29
<211>  704
<212>  PRT
<213>  Homo Sapiens

<400>  29

Met Glu Leu Arg Pro Trp Leu Leu Trp Val Val Ala Ala Thr Gly Thr
1               5                   10                  15

Leu Val Leu Leu Ala Ala Asp Ala Gln Gly Gln Lys Val Phe Thr Asn
            20                  25                  30

Thr Trp Ala Val Arg Ile Pro Gly Gly Pro Ala Val Ala Asn Ser Val
        35                  40                  45

Ala Arg Lys His Gly Phe Leu Asn Leu Gly Gln Ile Phe Gly Asp Tyr
    50                  55                  60

Tyr His Phe Trp His Arg Gly Val Thr Lys Arg Ser Leu Ser Pro His
65                  70                  75                  80

Arg Pro Arg His Ser Arg Leu Gln Arg Glu Pro Gln Val Gln Trp Leu
                85                  90                  95

Glu Gln Gln Val Ala Lys Arg Arg Thr Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Ala Ala Trp Ala Gln Gly Tyr Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Thr Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640

Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

<210>  30
<211>  666
<212>  PRT
<213>  Homo Sapiens

<400>  30

Tyr Asn Ser Gly Lys Leu Glu Glu Phe Val Gln Gly Asn Leu Glu Arg

1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val
        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile

275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys
385                 390                 395                 400

Val Ser Arg Tyr Val Asn Trp Ile Lys Glu Lys Thr Lys Leu Thr Ser
                405                 410                 415

Val Ser Gln Thr Ser Lys Leu Thr Arg Ala Glu Thr Val Phe Pro Asp
            420                 425                 430

Val Asp Glu Pro Lys Ser Cys Asp Lys Thr His Thr Cys Pro Pro Cys
        435                 440                 445

Pro Ala Pro Glu Leu Leu Gly Gly Pro Ser Val Phe Leu Phe Pro Pro
    450                 455                 460

Lys Pro Lys Asp Thr Leu Met Ile Ser Arg Thr Pro Glu Val Thr Cys
465                 470                 475                 480

Val Val Val Asp Val Ser His Glu Asp Pro Glu Val Lys Phe Asn Trp
                485                 490                 495

Tyr Val Asp Gly Val Glu Val His Asn Ala Lys Thr Lys Pro Arg Glu
            500                 505                 510

Glu Gln Tyr Asn Ser Thr Tyr Arg Val Val Ser Val Leu Thr Val Leu
        515                 520                 525

His Gln Asp Trp Leu Asn Gly Lys Glu Tyr Lys Cys Lys Val Ser Asn
    530                 535                 540

Lys Ala Leu Pro Ala Pro Ile Glu Lys Thr Ile Ser Lys Ala Lys Gly

545                 550                 555                 560

Gln Pro Arg Glu Pro Gln Val Tyr Thr Leu Pro Pro Ser Arg Glu Glu
                565                 570                 575

Met Thr Lys Asn Gln Val Ser Leu Thr Cys Leu Val Lys Gly Phe Tyr
            580                 585                 590

Pro Ser Asp Ile Ala Val Glu Trp Glu Ser Asn Gly Gln Pro Glu Asn
        595                 600                 605

Asn Tyr Lys Thr Thr Pro Pro Val Leu Asp Ser Asp Gly Ser Phe Phe
    610                 615                 620

Leu Tyr Ser Lys Leu Thr Val Asp Lys Ser Arg Trp Gln Gln Gly Asn
625                 630                 635                 640

Val Phe Ser Cys Ser Val Met His Glu Ala Leu His Asn His Tyr Thr
                645                 650                 655

Gln Lys Ser Leu Ser Leu Ser Pro Gly Lys
            660                 665

SEQUENCE LISTING

<110>  ZLB Behring GmbH

<120>  Proteolytically cleavable fusion proteins with high molar
       specific activity

<130>  2006_M004_A115

<160>  30

<170>  PatentIn version 3.3

<210>  1
<211>  585
<212>  PRT
<213>  Homo sapiens

<400>  1

<210>  2
<211>  415
<212>  PRT
<213>  Homo sapiens

<400>  2

Tyr Asn Ser Gly Lys Leu Glu Glu Phe Val Gln Gly Asn Leu Glu Arg
1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val

        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile
        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys

385                 390                 395                 400

Val Ser Arg Tyr Val Asn Trp Ile Lys Glu Lys Thr Lys Leu Thr
                405                 410                 415

<210>  3
<211>  1386
<212>  DNA
<213>  Homo sapiens

<400>  3
atgcagcgcg tgaacatgat catggcagaa tcaccaggcc tcatcaccat ctgcctttta      60

ggatatctac tcagtgctga atgtacagtt tttcttgatc atgaaaacgc caacaaaatt     120

ctgaatcggc caaagaggta taattcaggt aaattggaag agtttgttca agggaacctt     180

gagagagaat gtatggaaga aaagtgtagt tttgaagaag cacgagaagt ttttgaaaac     240

actgaaagaa caactgaatt ttggaagcag tatgttgatg gagatcagtg tgagtccaat     300

ccatgtttaa atggcggcag ttgcaaggat gacattaatt cctatgaatg ttggtgtccc     360

tttggatttg aaggaaagaa ctgtgaatta gatgtaacat gtaacattaa gaatggcaga     420

tgcgagcagt tttgtaaaaa tagtgctgat aacaaggtgg tttgctcctg tactgaggga     480

tatcgacttg cagaaaacca gaagtcctgt gaaccagcag tgccatttcc atgtggaaga     540

gtttctgttt cacaaacttc taagctcacc cgtgctgaga ctgtttttcc tgatgtggac     600

tatgtaaatt ctactgaagc tgaaaccatt ttggataaca tcactcaaag cacccaatca     660

tttaatgact tcactcgggt tgttggtgga gaagatgcca aaccaggtca attcccttgg     720

caggttgttt tgaatggtaa agttgatgca ttctgtggag gctctatcgt taatgaaaaa     780

tggattgtaa ctgctgccca ctgtgttgaa actggtgtta aaattacagt tgtcgcaggt     840

gaacataata ttgaggagac agaacataca gagcaaaagc gaaatgtgat tcgaattatt     900

cctcaccaca actacaatgc agctattaat aagtacaacc atgacattgc ccttctggaa     960

ctggacgaac ccttagtgct aaacagctac gttacaccta tttgcattgc tgacaaggaa    1020

tacacgaaca tcttcctcaa atttggatct ggctatgtaa gtggctgggg aagagtcttc    1080

cacaaaggga gatcagcttt agttcttcag taccttagag ttccacttgt tgaccgagcc    1140

acatgtcttc gatctacaaa gttcaccatc tataacaaca tgttctgtgc tggcttccat    1200

gaaggaggta gagattcatg tcaaggagat agtgggggac cccatgttac tgaagtggaa    1260

gggaccagtt tcttaactgg aattattagc tggggtgaag agtgtgcaat gaaaggcaaa    1320

tatggaatat ataccaaggt atcccggtat gtcaactgga ttaaggaaaa aacaaagctc    1380

acttaa                                                               1386

<210>  4
<211>  1830
<212>  DNA
<213>  Homo sapiens

<400>  4

atgaagtggg taacctttat ttcccttctt tttctcttta gctcggctta ttccaggggt 60
gtgtttcgtc gagatgcaca caagagtgag gttgctcatc ggtttaaaga tttgggagaa 120
gaaaatttca aagccttggt gttgattgcc tttgctcagt atcttcagca gtgtccattt 180
gaagatcatg taaaattagt gaatgaagta actgaatttg caaaaacatg tgttgctgat 240
gagtcagctg aaaattgtga caaatcactt catacccttt ttggagacaa attatgcaca 300
gttgcaactc ttcgtgaaac ctatggtgaa atggctgact gctgtgcaaa acaagaacct 360
gagagaaatg aatgcttctt gcaacacaaa gatgacaacc caaacctccc ccgattggtg 420
agaccagagg ttgatgtgat gtgcactgct tttcatgaca atgaagagac atttttgaaa 480
aaatacttat atgaaattgc cagaagacat ccttactttt atgccccgga actccttttc 540
tttgctaaaa ggtataaagc tgcttttaca gaatgttgcc aagctgctga taaagctgcc 600
tgcctgttgc caaagctcga tgaacttcgg gatgaaggga aggcttcgtc tgccaaacag 660
agactcaagt gtgccagtct ccaaaaattt ggagaaagag ctttcaaagc atgggcagta 720
gctcgcctga gccagagatt tcccaaagct gagtttgcag aagtttccaa gttagtgaca 780
gatcttacca aagtccacac ggaatgctgc catggagatc tgcttgaatg tgctgatgac 840
agggcggacc ttgccaagta tatctgtgaa aatcaagatt cgatctccag taaactgaag 900
gaatgctgtg aaaaacctct gttggaaaaa tcccactgca ttgccgaagt ggaaaatgat 960
gagatgcctg ctgacttgcc ttcattagct gctgattttg ttgaaagtaa ggatgtttgc 1020
aaaaactatg ctgaggcaaa ggatgtcttc ctgggcatgt ttttgtatga atatgcaaga 1080
aggcatcctg attactctgt cgtgctgctg ctgagacttg ccaagacata tgaaaccact 1140
ctagagaagt gctgtgccgc tgcagatcct catgaatgct atgccaaagt gttcgatgaa 1200
tttaaacctc ttgtggaaga gcctcagaat ttaatcaaac aaaattgtga gctttttgag 1260
cagcttggag agtacaaatt ccagaatgcg ctattagttc gttacaccaa gaaagtaccc 1320
caagtgtcaa ctccaactct tgtagaggtc tcaagaaacc taggaaaagt gggcagcaaa 1380
tgttgtaaac atcctgaagc aaaaagaatg ccctgtgcag aagactatct atccgtggtc 1440
ctgaaccagt tatgtgtgtt gcatgagaaa acgccagtaa gtgacagagt caccaaatgc 1500
tgcacagaat ccttggtgaa caggcgacca tgcttttcag ctctggaagt cgatgaaaca 1560
tacgttccca aagagtttaa tgctgaaaca ttcaccttcc atgcagatat atgcacactt 1620
tctgagaagg agagacaaat caagaaacaa actgcacttg ttgagctcgt gaaacacaag 1680
cccaaggcaa caaaagagca actgaaagct gttatggatg atttcgcagc ttttgtagag 1740
aagtgctgca aggctgacga taaggagacc tgctttgccg aggagggtaa aaaacttgtt 1800
gctgcaagtc aagctgcctt aggcttataa 1830

<210> 5
<211> 21
<212> DNA
<213> Artificial Sequence

<220>
<223>  Artificial

<400>  5
ccactttcac aatctgctag c                                                21


<210>  6
<211>  23
<212>  DNA
<213>  Artificial Sequence

<220>
<223>  Artificial

<400>  6
caattccaat gaattaacct tgg                                              23


<210>  7
<211>  21
<212>  DNA
<213>  Artificial Sequence

<220>
<223>  Artificial

<400>  7
atgcagcgcg tgaacatgat c                                                21


<210>  8
<211>  25
<212>  DNA
<213>  Artificial Sequence

<220>
<223>  Artificial

<400>  8
tcattaagtg agctttgttt tttcc                                            25


<210>  9
<211>  21
<212>  DNA
<213>  Artificial Sequence

<220>
<223>  Artificial

<400>  9
gattcgaatt cgcccttatg c                                                21


<210>  10
<211>  32
<212>  DNA
<213>  Artificial Sequence

<220>
<223>  Artificial

<400>  10
cgctcgaggt gagctttgtt ttttccttaa tc                                    32


<210>  11

<211>  52
<212>  DNA
<213>  Artificial Sequence

<220>
<223>  Artificial

<400>  11
ctcgagcggg ggatctggcg ggtctggagg ctctggaggg tcgggaggct ct          52


<210>  12
<211>  46
<212>  DNA
<213>  Artificial Sequence

<220>
<223>  Artificial

<400>  12
ggatccagat cccccagagc ctccagagcc tcccgaccct ccagag          46


<210>  13
<211>  56
<212>  DNA
<213>  Artificial Sequence

<220>
<223>  Artificial

<400>  13
ctcgagcaat ggatctggcg ggtctggagg ctctggaggg tcgaatggct ctggag          56


<210>  14
<211>  64
<212>  DNA
<213>  Artificial Sequence

<220>
<223>  Artificial

<400>  14
ggatccgttc cctccagacc cgccagatcc cccagagcct ccagagccat tcgaccctcc          60

agag          64


<210>  15
<211>  46
<212>  DNA
<213>  Artificial Sequence

<220>
<223>  Artificial

<400>  15
cctcgagctc tgtgagccag acctccaagc tcaccagggc cgagac          46


<210>  16
<211>  44
<212>  DNA
<213>  Artificial Sequence

<220>
<223>  Artificial

<400>  16
gggatccgtc cacatcaggg aagacagtct cggccctggt gagc                        44

<210>  17
<211>  33
<212>  DNA
<213>  Artificial Sequence

<220>
<223>  Artificial

<400>  17
ggaaaaaaca aagctcactt ctgtgagcca gac                                    33

<210>  18
<211>  33
<212>  DNA
<213>  Artificial Sequence

<220>
<223>  Artificial

<400>  18
gtctggctca cagaagtgag ctttgttttt tcc                                    33

<210>  19
<211>  38
<212>  DNA
<213>  Artificial Sequence

<220>
<223>  Artificial

<400>  19
cctcgagcag agcttcaatg acttcacccg ggtggtgg                               38

<210>  20
<211>  41
<212>  DNA
<213>  Artificial Sequence

<220>
<223>  Artificial

<400>  20
gggatccatc ctccccgccc accacccggg tgaagtcatt g                           41

<210>  21
<211>  34
<212>  DNA
<213>  Artificial Sequence

<220>
<223>  Artificial

<400>  21
ggaaaaaaca aagctcactc agagcttcaa tgac                                   34

<210>  22
<211>  34
<212>  DNA

<213>  Artificial Sequence

<220>
<223>  Artificial

<400>  22
gtcattgaag ctctgagtga gctttgtttt ttcc                                    34


<210>  23
<211>  31
<212>  DNA
<213>  Artificial Sequence

<220>
<223>  Artificial

<400>  23
gtgggatccg atgcacacaa gagtgaggtt g                                       31


<210>  24
<211>  35
<212>  DNA
<213>  Artificial Sequence

<220>
<223>  Artificial

<400>  24
cacggatccc tataagccta aggcagcttg acttg                                   35


<210>  25
<211>  18
<212>  DNA
<213>  Artificial Sequence

<220>
<223>  Artificial

<400>  25
caaggagacg ggcgctcc                                                      18


<210>  26
<211>  19
<212>  DNA
<213>  Artificial Sequence

<220>
<223>  Artificial

<400>  26
gcccaaggag gggattggc                                                     19


<210>  27
<211>  30
<212>  DNA
<213>  Artificial Sequence

<220>
<223>  Artificial

<400>  27
gtggaattca tggagctgag gccctggttg                                         30

<210> 28
<211> 38
<212> DNA
<213> Artificial Sequence

<220>
<223> Artificial

<400> 28
cacgcggccg ctcactacag ccgttgcccc gcctccac 38


<210> 29
<211> 704
<212> PRT
<213> Homo Sapiens

<400> 29

Met Glu Leu Arg Pro Trp Leu Leu Trp Val Val Ala Ala Thr Gly Thr
1               5                   10                  15

Leu Val Leu Leu Ala Ala Asp Ala Gln Gly Gln Lys Val Phe Thr Asn
            20                  25                  30

Thr Trp Ala Val Arg Ile Pro Gly Gly Pro Ala Val Ala Asn Ser Val
        35                  40                  45

Ala Arg Lys His Gly Phe Leu Asn Leu Gly Gln Ile Phe Gly Asp Tyr
    50                  55                  60

Tyr His Phe Trp His Arg Gly Val Thr Lys Arg Ser Leu Ser Pro His
65                  70                  75                  80

Arg Pro Arg His Ser Arg Leu Gln Arg Glu Pro Gln Val Gln Trp Leu
                85                  90                  95

Glu Gln Gln Val Ala Lys Arg Arg Thr Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Ala Ala Trp Ala Gln Gly Tyr Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Thr Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640

Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

<210>  30
<211>  666
<212>  PRT
<213>  Homo Sapiens

<400>  30

Tyr Asn Ser Gly Lys Leu Glu Glu Phe Val Gln Gly Asn Leu Glu Arg

1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val
        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile

        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys
385                 390                 395                 400

Val Ser Arg Tyr Val Asn Trp Ile Lys Glu Lys Thr Lys Leu Thr Ser
                405                 410                 415

Val Ser Gln Thr Ser Lys Leu Thr Arg Ala Glu Thr Val Phe Pro Asp
            420                 425                 430

Val Asp Glu Pro Lys Ser Cys Asp Lys Thr His Thr Cys Pro Pro Cys
        435                 440                 445

Pro Ala Pro Glu Leu Leu Gly Gly Pro Ser Val Phe Leu Phe Pro Pro
    450                 455                 460

Lys Pro Lys Asp Thr Leu Met Ile Ser Arg Thr Pro Glu Val Thr Cys
465                 470                 475                 480

Val Val Val Asp Val Ser His Glu Asp Pro Glu Val Lys Phe Asn Trp
                485                 490                 495

Tyr Val Asp Gly Val Glu Val His Asn Ala Lys Thr Lys Pro Arg Glu
            500                 505                 510

Glu Gln Tyr Asn Ser Thr Tyr Arg Val Val Ser Val Leu Thr Val Leu
        515                 520                 525

His Gln Asp Trp Leu Asn Gly Lys Glu Tyr Lys Cys Lys Val Ser Asn
    530                 535                 540

Lys Ala Leu Pro Ala Pro Ile Glu Lys Thr Ile Ser Lys Ala Lys Gly

545                 550                 555                 560

Gln Pro Arg Glu Pro Gln Val Tyr Thr Leu Pro Pro Ser Arg Glu Glu
                565                 570                 575

Met Thr Lys Asn Gln Val Ser Leu Thr Cys Leu Val Lys Gly Phe Tyr
            580                 585                 590

Pro Ser Asp Ile Ala Val Glu Trp Glu Ser Asn Gly Gln Pro Glu Asn
        595                 600                 605

Asn Tyr Lys Thr Thr Pro Pro Val Leu Asp Ser Asp Gly Ser Phe Phe
    610                 615                 620

Leu Tyr Ser Lys Leu Thr Val Asp Lys Ser Arg Trp Gln Gln Gly Asn
625                 630                 635                 640

Val Phe Ser Cys Ser Val Met His Glu Ala Leu His Asn His Tyr Thr
                645                 650                 655

Gln Lys Ser Leu Ser Leu Ser Pro Gly Lys
            660                 665

## Description

### Introduction

The present invention relates to the field of modified therapeutic fusion proteins with increased half-life compared to their non-modified parent therapeutic polypeptides. The invention specifically relates to coagulation factors fused to half-life enhancing polypeptides (HLEPs), which are connected by linker peptides that are proteolytically cleavable. The cleavage of such linkers liberates the therapeutic polypeptide from any activity-compromising steric hindrance caused by the HLEP and, thereby, allows the generation of fusion proteins, which retain a high molar specific activity of the coagulation factor. In case the therapeutic fusion proteins are zymogens, those linkers are especially preferred that liberate the therapeutic polypeptide essentially simultaneous to its activation in vivo upon exposure to the corresponding protease(s). Another aspect of the present invention is a faster inactivation and/or elimination rate of said coagulation factors once they are proteolytically cleaved respectively activated compared to the corresponding fusion proteins without cleavable linker.

The idea of the invention is demonstrated in particular by human vitamin K-dependent polypeptides Factor IX, Factor VII, and Factor VIIa but the concept also may be applied to other coagulation factors. Any half-life enhancing polypeptide (HLEP) may be connected to the therapeutic polypeptide by a cleavable linker peptide, but albumin or immunoglobulines or fragments derived thereof like the Fc fragment without an antigen binding domain are preferred HLEPs. The invention also relates to cDNA sequences coding for the therapeutic polypeptides and derivatives thereof genetically fused to a cDNA coding for HLEPs like human serum albumin linked by oligonucleotides which code for cleavable, intervening peptidic linkers, such encoded derivatives exhibiting improved half life and almost uncompromised molar specific activity, recombinant expression vectors containing such cDNA sequences, host cells transformed with such recombinant expression vectors, recombinant polypeptides and derivatives which do have biological activities comparable to the unmodified wild type therapeutic polypeptide but having improved half-life and processes for the manufacture of such recombinant proteins and their derivatives. The invention also covers a transfer vector for use in human gene therapy, which comprises such modified DNA sequences useful to increase half-life in vivo.

### Background of the invention

Several recombinant, therapeutic polypeptides are meanwhile commercially available for therapeutic and prophylactic use in humans. The patients in general benefit from the specific mode of action of the recombinant active ingredients but a disadvantage often is their limited availability due to their expensive and complex manufacturing processes. A reduction of the necessary dose or the frequency of application of such products could improve this situation. A reduced frequency of application in addition could improve the convenience for the patient and, therefore, also the acceptance of the therapy. Several solutions were described to achieve the goal of an increased in vivo half-life after application. Solutions proposed recently were the formation of fusion proteins, especially in the case of polypeptides with a short in vivo half-life that can be increased significantly by fusion to a HLEP.

Ballance et al. (WO 01/79271) described fusion polypeptides of a multitude of different therapeutic polypeptides which, when fused to human serum albumin, are predicted to have an increased functional half-life in vivo and extended shelf-life. Long lists of potential fusion partners are described without showing by experimental data for almost any of these polypeptides that the respective albumin fusion proteins actually retain biological activity and have improved properties. Among said list of therapeutic polypeptides also Factor IX and FVII/FVIIa are mentioned as examples of the invention. Also described are fusions of FIX and FVII/FVIIa in which there is a peptidic linker between albumin and FIX or FVII/FVIIa. However, the use of cleavable linker peptides is not suggested.

Furthermore, Sheffield et al. (Sheffield W.P. et al. (2004), Br. J. Haematol. 126: 565-573) expressed a murine Factor IX albumin fusion protein composed of murine FIX, a linker of 8 amino acids (GPG₄TM), murine albumin and a peptide tag of 22 amino acids as well as a human Factor IX albumin fusion protein composed of human Factor IX, a linker of 7 amino acids (G₆V) and human albumin. Using a one-stage, FIX dependent clotting assay, the molar specific activities of the murine FIX-albumin fusion protein (MFUST) and the human FIX-albumin fusion protein (HFUS) were at least two- to three-fold lower than that of their unfused counterparts, an effect attributed at least partially to a slower proteolytic activation process by FXIa. Sheffield did not use or suggest to use a cleavable linker between FIX and albumin.

Several patent applications describe the fusion of therapeutic polypeptides to immunoglobulin constant regions to extend the therapeutic polypeptide's in vivo half-life. WO 2002/04598, WO 2003/059935, WO 2004/081053, WO 2004/101740 and WO 2005/001025 include FIX as examples for the therapeutic polypeptide moiety. The latter two patent applications also describe FVII/FVIIa fusions to immunoglobulin constant regions and find that fusion protein homodimers have inferior clotting activity compared to fusion proteins consisting of a monomer/dimer. Again, the use of cleavable linker peptides is not suggested.

In WO 91/09125 fusion proteins are disclosed that are joined by linkers which are cleavable by proteases of the blood coagulation cascade, but said fusion proteins are limited to those comprising fibrinolytic or antithrombotic proteins.

In WO 03/068934 chimeric molecules are described, that are composed of at least one first component molecule, at least one linker and at least one second molecule,
wherein the linker comprises an enzyme cleavage site to produce a non-naturally occurring linkage and cleavage site between the first and the second component molecule and wherein, upon cleavage of the chimeric molecule at the cleavage site, at least one of the component molecules is functionally active. The cleaving proteases may be coagulation factors like thrombin. Component molecules described among many others are FIX and FVIIa. However the therapeutic fusion proteins of the present invention are not disclosed individually, nor are improved properties of the therapeutic fusion proteins of the present invention disclosed, like increased molar specific activity, or increased inactivation and/or elimination rates as compared to the therapeutic protein without cleavable linkers.

### Description of the invention

There is a high medical need for coagulation factors which do have a long half-life. In the prior art fusions of coagulation factors to half-life enhancing polypeptides have been suggested to achieve this goal. However, once a coagulation factor is activated during coagulation either by proteolytic cleavage of the zymogen (like FIX) or by contact of an already proteolytically "pre"-activated factor to a second polypeptide (like FVIIa binding to Tissue Factor), it is no longer desirable to maintain the long half-life of the now activated coagulation factor as this might lead to thrombotic complications what is already the case for a wild type coagulation factor as FVIIa (Aledort L.M., J Thromb Haemost 2(10): 1700-1708 (2004)) and what should be much more relevant if the activated factor would have an increased half-life. It is therefore one objective of the present invention to provide long-lived coagulation factors, which after activation or after availability of a cofactor do have a half-life comparable to that of an unmodified coagulation factor.

Fusions of the coagulation factors to half-life enhancing polypeptides as described in the prior art suffer in general from a reduced molar specific activity of the fused coagulation factor. It is another aspect of the present invention to provide coagulation factors with enhanced half-life, which do show increased molar specific activity compared to the corresponding therapeutic fusion protein without cleavable linker.

The invention is therefore about therapeutic fusion proteins comprising
a) a coagulation factor, its variants or derivatives,
b) a half-life enhancing polypeptide selected from the group consisting of albumin including variants and derivatives thereof and immunoglobulins including variants and derivatives thereof and
c) a peptidic linker which joins the coagulation factor and the half-life enhancing polypeptide;
wherein the peptidic linker is cleavable by proteases involved in coagulation or activated by coagulation enzymes and the therapeutic fusion protein has in comparison to the respective therapeutic fusion protein without cleavable linker
i) an increased molar specific activity of the coagulation factor and/or
ii) an increased inactivation rate of the coagulation factor after its cleavage respectively activation and/or
iii) an increased elimination rate of the coagulation factor after proteolytical cleavage of the therapeutic fusion protein.

As a consequence of the cleavable linker, after cleavage respectively activation, the coagulation factor more closely resembles the behaviour of the native, non-fused factor and does not show an increased half-life of the active factor with potentially prothrombotic effect.

A further aspect of the present invention is to provide therapeutic fusion proteins comprising
a) a coagulation factor, its variants or derivatives,
b) a half-life enhancing polypeptide selected from the group consisting of albumin including variants and derivatives thereof and immunoglobulins including variants and derivatives thereof and
c) a peptidic linker which joins the coagulation factor and the half-life enhancing polypeptide;
wherein the peptidic linker is cleavable by proteases involved in coagulation or activated by coagulation enzymes and the therapeutic fusion protein has in comparison to the respective therapeutic fusion protein without cleavable linker
i) an increased molar specific activity of the coagulation factor and/or
ii) an increased inactivation rate of the coagulation factor after its cleavage respectively activation and/or
iii) an increased elimination rate of the coagulation factor after proteolytical cleavage of the therapeutic fusion protein
and which do have an enhanced in vivo recovery as compared to the in vivo recovery of the unmodified coagulation factor.

Preferred are therapeutic fusion proteins which have an enhanced in vivo recovery as compared to the unmodified coagulation factor by at least 10%, more preferred by at least 25% and most preferred by 40% or more.

Preferred coagulation factors are vitamin-K dependent coagulation factors and fragments and variants thereof, even more preferred are FVIIa and FIX and fragments and variants thereof.

Preferred HLEPs are albumin and fragments or variants thereof and immunoglobulins site including fragments and variants thereof.

The linker region in a preferred embodiment comprises a sequence of the therapeutic polypeptide to be applied or a variant thereof, which results in a decreased risk of neoantigenicity formation of the construct and in an adaptation of the kinetics of the linker cleavage to the activation cleavage of the therapeutic fusion protein should this be a zymogen. Thus, in such preferred embodiments a zymogen and a corresponding linker are activated, respectively cleaved, with comparable kinetics. For this reason, the present invention particularly relates to fusion proteins of a zymogen and a HLEP, where the kinetics of the linker cleavage by relevant proteases is not delayed by more than a factor of 3, preferably not by more than a factor of 2 compared to the kinetics of the zymogen activation.

In a further embodiment, the linker peptide comprises cleavage sites for more than one protease. This can either be achieved by a linker peptide that can be cleaved at the same position by different proteases or by a linker peptide that provides two or more different cleavage sites. This may be of advantage at circumstances where the therapeutic fusion protein has to be activated for achieving enzymatic activity and where different proteases may contribute to this activation step. This e.g. is the case upon activation of FIX, which can either be achieved by FXIa or by FVIIa/Tissue Factor (TF).

Preferred embodiments of the invention are therapeutic fusion proteins wherein the linker is cleavable by the protease, which activates the coagulation factor, thereby ensuring that the cleavage of the linker is linked to the activation of the coagulation factor at a site at which coagulation occurs.

Other preferred therapeutic fusion proteins according to the invention are those,
wherein the linker is cleavable by a protease, which is activated upon involvement of the coagulation factor also ensuring that cleavage of the fusion protein is connected with a coagulatory event.

One class of most preferred therapeutic fusion proteins are those wherein the linker is cleavable by FXIa and/or by FVIIa/TF and the coagulation factor is FIX

The idea of the invention is demonstrated in particular by the vitamin K-dependent polypeptide Factor IX, cleavable linkers and albumin as the HLEP as well as its cDNA sequences. But the invention also relates to cDNA sequences coding for any other coagulation factors which can be proteolytically activated or that are involved in the activation of other zymogens or polypeptides and which are genetically fused to cDNA sequences coding for human serum albumin or other HLEPs, which are linked by oligonucleotides that code for intervening, cleavable peptide linkers, such encoded derivatives exhibiting almost comparable molar specific activity than their unmodified therapeutic polypeptides, recombinant expression vectors containing such cDNA sequences, host cells transformed with such recombinant expression vectors, recombinant therapeutic fusion proteins and derivatives that have biological activities almost comparable to the unmodified wild type therapeutic polypeptides but having improved in vivo half-life and processes for the manufacture of such recombinant polypeptides and their derivatives. The invention also covers a transfer vector for use in human gene therapy, which comprises such modified DNA sequences useful to increase product levels in vivo.

Preferred therapeutic fusion proteins according to the invention are those that do have a molar specific activity that is at least 25% increased compared to that of the therapeutic fusion protein without a cleavable linker. More preferred are therapeutic fusion proteins in which the molar specific activity is increased by 50%, even more preferred those in which the molar specific activity is increased by 100%.

Also preferred embodiments of the present invention are therapeutic fusion proteins, wherein the inactivation rate of the activated coagulation factor after cleavage of the peptidic linker which links the coagulation factor to the half-life enhancing polypeptide is increased by at least 10% as compared to the inactivation rate of the activated coagulation factor in a corresponding therapeutic fusion protein without a cleavable linker. More preferred are therapeutic fusion proteins in which the inactivation rate is increased by 25%, even more preferred those in which the inactivation rate is increased by 50%.

Further preferred embodiments of the present invention are therapeutic fusion proteins, wherein the elimination rate of the coagulation factor after cleavage of the peptidic linker which links the coagulation factor to the half-life enhancing polypeptide is increased by at least 10% as compared to the elimination rate of the coagulation factor in a corresponding therapeutic fusion protein without a cleavable linker. More preferred are therapeutic fusion proteins in which the elimination rate is increased by 25%, even more preferred those in which the elimination rate is increased by 50%.

### Detailed description of the invention

### Vitamin K-dependent polypeptides

Vitamin K-dependent polypeptides as one group of the therapeutic polypeptides are polypeptides that are γ-carboxylated enzymatically in the liver using vitamin K as a cofactor. Such vitamin K-dependent polypeptides e.g. are Factors II, VII, IX, X, Protein C, Protein S, GAS6, and Protein Z.

### Human FIX

Human FIX, one member of the group of vitamin K-dependent polypeptides, is a single-chain glycoprotein with a molecular weight of 57 kDa, which is secreted by liver cells into the blood stream as an inactive zymogen of 415 amino acids. It contains 12 γ-carboxy-glutamic acid residues localized in the N-terminal Gla-domain of the polypeptide. The Gla residues require vitamin K for their biosynthesis. Following the Gla domain there are two epidermal growth factor domains, an activation peptide, and a trypsin-type serine protease domain. Further posttranslational modifications of FIX encompass hydroxylation (Asp 64), N-(Asn157 and Asn167) as well as O-type glycosylation (Ser53, Ser61, Thr159, Thr169, and Thr172), sulfation (Tyr155), and phosphorylation (Ser158).
FIX is converted to its active form Factor IXa by proteolysis of the activation peptide at Arg145-Ala146 and Arg180-Val181 leading to the formation of two polypeptide chains, an N-terminal light chain (18 kDa) and a C-terminal heavy chain (28 kDa), which are held together by one disulfide bridge. Activation cleavage of Factor IX can be achieved in vitro e.g. by Factor Xla or Factor Vlla/TF. Factor IX is present in human plasma in a concentration of 5-10 µg/ml. Plasma half-life of Factor IX in humans was found to be about 15 to 18 hours ( White GC et al. 1997. Recombinant factor IX. Thromb Haemost. 78: 261-265; Ewenstein BM et al. 2002. Pharmacokinetic analysis of plasma-derived and recombinant F IX concentrates in previously treated patients with moderate or severe hemophilia B. Transfusion 42:190-197).

Hemophilia B is caused by non-functional or missing Factor IX and is treated with Factor IX concentrates from plasma or a recombinant form of Factor IX. As haemophilia B patients often receive at least biweekly prophylactic administrations of Factor IX to avoid spontaneous bleedings, it is desirable to increase the intervals of application by increasing the half-life of the F IX product applied. An improvement in plasma half-life would bring significant benefit to the patient. Up to now no pharmaceutical preparation of a Factor IX with improved plasma half-life is commercially available nor have any data been published showing F IX variants with prolonged in vivo half-life and almost unchanged molar specific activity. Therefore, a high medical need still exists to develop forms of Factor IX which have a longer functional half-life in vivo.

### Factor VII and Factor VIIa

FVII is a single-chain glycoprotein with a molecular weight of 50 kDa, which is secreted by liver cells into the blood stream as an inactive zymogen of 406 amino acids. FVII is converted to its active form Factor VIIa by proteolysis of the single peptide bond at Arg152-IIe 153 leading to the formation of two polypeptide chains, a N-terminal light chain (24 kDa) and a C-terminal heavy chain (28 kDa), which are held together by one disulfide bridge. In contrast to other vitamin K-dependent coagulation factors, no activation peptide is cleaved off during activation. Activation cleavage of Factor VII can be achieved in vitro e.g. by Factor Xa, Factor Xlla, Factor IXa, Factor VIIa, Factor Seven Activating Protease (FSAP), and thrombin.
Mollerup et al. (Biotechnol. Bioeng. (1995) 48: 501-505) reported that some cleavage also occurs in the heavy chain at Arg290 and or Arg315.

Factor VII is present in plasma in a concentration of 500 ng/ml. About 1% or 5 ng/ml of Factor VII are present as Factor VIIa. Plasma half-life of Factor VII was found to be about 4 hours and that of Factor VIIa about 2 hours.

It was shown that by administering supraphysiological concentrations of Factor VIIa hemostasis can be achieved bypassing the need for Factor VIIIa and Factor IXa. The cloning of the cDNA for Factor VII (US 4,784,950) made it possible to develop activated Factor VII as a pharmaceutical. Factor VIIa was successfully administered for the first time in 1988. Ever since the number of indications of Factor VIIa has grown steadily showing a potential to become an universal hemostatic agent to stop bleeding (Erhardtsen, 2002). However, the short half-life of Factor VIIa of approximately 2 hours and reduced in vivo recovery is limiting its application. Therefore, a high medical need still exists to develop forms of Factor VIIa which have an improved half-life but otherwise uncompromised molar specific activity and inactivation kinetics.

### Therapeutic fusion proteins

"Therapeutic fusion proteins" in the sense of this invention are coagulation factors fused to a HLEP that upon application to a human or animal can produce a prophylactic or therapeutic effect. These therapeutic fusion proteins may be applied to a human or an animal via oral, topical, parenteral or other routes. Specific classes of therapeutic fusion proteins covered, i.e. by the examples in this invention, are coagulation factors like e.g. vitamin K-dependent polypeptides linked to half-life enhancing polypeptides like e.g. albumin and immunoglobulines and their derivatives. The expression "therapeutic fusion protein" is used interchangeable with "fusion protein".

### Half-life enhancing polypeptide (HLEP)

As half-life enhancing polypeptides (HLEPs) albumin and immunoglobulines and their fragments or derivatives have been described. The terms, human serum albumin (HSA) and human albumin (HA) are used interchangeably in this application. The terms "albumin" and "serum albumin" are broader, and encompass human serum albumin (and fragments and variants thereof) as well as albumin from other species (and fragments and variants thereof).

As used herein, "albumin" refers collectively to albumin polypeptide or amino acid sequence, or an albumin fragment or variant, having one or more functional activities (e.g., biological activities) of albumin. In particular, "albumin" refers to human albumin or fragments thereof, especially the mature form of human albumin as shown in SEQ ID No:1 herein or albumin from other vertebrates or fragments thereof, or analogs or variants of these molecules or fragments thereof.

The albumin portion of the albumin fusion proteins may comprise the full length of the HA sequence as described above, or may include one or more fragments thereof that are capable of stabilizing or prolonging the therapeutic activity. Such fragments may be of 10 or more amino acids in length or may include about 15, 20, 25, 30, 50, or more contiguous amino acids from the HA sequence or may include part or all of specific domains of HA.

The albumin portion of the albumin fusion proteins of the invention may be a variant of normal HA. The therapeutic polypeptide portion of the fusion proteins of the invention may also be variants of the corresponding therapeutic polypeptides as described herein. The term "variants" includes insertions, deletions and substitutions, either conservative or non-conservative, where such changes do not substantially alter the active site, or active domain which confers the therapeutic activities of the therapeutic polypeptides.

In particular, the albumin fusion proteins of the invention may include naturally occurring polymorphic variants of human albumin and fragments of human albumin. The albumin may be derived from any vertebrate, especially any mammal, for example human, cow, sheep, or pig. Non-mammalian albumins include, but are not limited to, hen and salmon. The albumin portion of the albumin-linked polypeptide may be from a different animal than the therapeutic polypeptide portion.

Generally speaking, an albumin fragment or variant will be at least 10, preferably at least 40, most preferably more than 70 amino acids long. The albumin variant may preferentially consist of or alternatively comprise at least one whole domain of albumin or fragments of said domains, for example domains 1 (amino acids 1-194 of SEQ ID NO:1), 2 (amino acids 195-387 of SEQ ID NO: 1), 3 (amino acids 388-585 of SEQ ID NO: 1), 1 + 2 (1-387 of SEQ ID NO: 1), 2 + 3 (195-585 of SEQ ID NO: 1) or 1 + 3 (amino acids 1-194 of SEQ ID NO: 1 + amino acids 388-585 of SEQ ID NO: 1). Each domain is itself made up of two homologous subdomains namely 1-105, 120-194, 195-291, 316-387, 388-491 and 512-585, with flexible inter-subdomain linker regions comprising residues Lys106 to Glu119, Glu292 to Val315 and Glu492 to Ala511.

The albumin portion of an albumin fusion protein of the invention may comprise at least one subdomain or domain of HA or conservative modifications thereof.

IgG and lgG-fragments may also be used as HLEPs. The therapeutic polypeptide portion is connected to the lgG or the lgG fragments via a cleavable linker that allows high molar specific activities of the fusion protein. Examples for factor VII/VIIa and factor IX lgG fusion molecules are found e.g. in WO 2005/001025 which is incorporated herein by reference in its entirety. It discloses i.e. a homodimer comprised of two factor VII molecules and two Fc molecules and a monomer/dimer hybrid comprised of one FVII molecule and two Fc molecules, the monomer/dimer showing an about four times greater clotting activity than the homodimer. A linker sequence of the present invention liberating the FVII molecules upon cleavage by a protease of the coagulation cascade like e.g. FXIa could be able to elevate the clotting activity of the constructs and especially that of the homodimer to an activity level comparable to the monomer/dimer or even higher. A FIX-Fc fusion protein with cleavable linker is exemplarily shown in SEQ ID No 93.

Cleavable linkers such as e.g. shown in table 3a and 3b may be applied in this case.

The invention specifically relates to fusion proteins, comprising linking a coagulation factor or fragment or variant thereof to the N- or C-terminus of a HLEP or fragment or variant thereof such that an intervening cleavable peptidic linker is introduced between the therapeutic polypeptide and the HLEP such that the fusion protein formed has an increased in vivo half-life compared to the coagulation factor which has not been linked to a HLEP and that the fusion protein has an at least 25% higher molar specific activity compared to the corresponding fusion protein with non-cleavable linker.

"Coagulation factor" as used in this application include, but is not limited to, polypeptides consisting of Factor IX, Factor VII, Factor VIII, von Willebrand Factor, Factor V, von Willebrand factor, Factor X, Factor XI, Factor XII, Factor XIII, Factor I, Factor II (Prothrombin), Protein C, Protein S, GAS6, or Protein Z as well as their activated forms. Furthermore, useful therapeutic polypeptides may be wild-type polypeptides or may contain mutations. Degree and location of glycosylation or other post-translation modifications may vary depending on the chosen host cells and the nature of the host cellular environment. When referring to specific amino acid sequences, posttranslational modifications of such sequences are encompassed in this application.

"Coagulation factor" within the above definition includes polypeptides that have the natural amino acid sequence including any natural polymorphisms. It also includes polypeptides with a slightly modified amino acid sequence, for instance, a modified N-terminal or C-terminal end including terminal amino acid deletions or additions as long as those polypeptides substantially retain the activity of the respective therapeutic polypeptide. Variants included differ in one or more amino acid residues from the wild type sequence. Examples of such differences may include truncation of the N- and/or C-terminus by one or more amino acid residues (e.g. preferably 1 to 30 amino acid residues), or addition of one or more extra residues at the N-and/or C-terminus, as well as conservative amino acid substitutions, i.e. substitutions performed within groups of amino acids with similar characteristics, e.g. (1) small amino acids, (2) acidic amino acids, (3) polar amino acids, (4) basic amino acids, (5) hydrophobic amino acids, and (6) aromatic amino acids. Examples of such conservative substitutions are shown in the following table.

**Table 1**

| | | | | |
|---|---|---|---|---|
| (1) | Alanine | Glycine | | |
| (2) | Aspartic acid | Glutamic acid | | |
| (3a) | Asparagine | Glutamine | | |
| (3b) | Serine | Threonine | | |
| (4) | Arginine | Histidine | Lysine | |
| (5) | Isoleucine | Leucine | Methionine | Valine |
| (6) | Phenylalanine | Tyrosine | Tryptophane | |

The in vivo half-life of the fusion proteins of the invention is usually at least about 50%, preferred more than 100% higher than the in vivo half-life of the non-fused polypeptide.

The fusion proteins of the present invention have at least a 25%, preferably at least a 50%, more preferably a 100% increased molar specific activity or biologic activity compared to the corresponding fusion proteins without cleavable linkers.

The molar specific activity in this regard is defined as the activity expressed per mole (or nmole) of the therapeutic polypeptide or therapeutic fusion protein of interest. Calculation of the molar specific activity allows a direct comparison of the activity of the different constructs which is not affected by the different molecular weights or optical densities of the polypeptides studied. The molar specific activity may be calculated as exemplified in table 2 below for FIX and a FIX-HSA fusion protein.

**Table 2: Calculation of molar specific activity as shown for a purified FIX-HSA fusion protein**

| Product | OD_{(280nm, 1%)} | MW | Activity/OD₂₈₀ (IU/L/OD₂₈₀) | Molar optical density (OD₍₂₈₀₎ at 1 mol/L) | Calculation of molar specific activity (IU/mol/L) |
|---|---|---|---|---|---|
| FIX | 13.3¹⁾ | 57000 | determined for product | 75810 (= MW x OD_{(280, 1%)}/10) | =(Activity/OD₂₈₀) x (OD₂₈₀ at 1 mol/L) |
| HSA | 5.7 ²⁾ | 66 300 | | 37791 (= MW x OD_{(280, 1%)}/10) | |
| FIX-HSA | | | determined for product | 113601 (= sum of molar optical density of FIX and HSA) | = (Activity/OD₂₈₀) x (OD₂₈₀ at 1 mol/L) |

| | | | | | |
|---|---|---|---|---|---|
| ¹⁾ R.G. Di Scipio et al., Biochem. 16: 698-706 (1977) ²⁾ C. Chaudhury et al, J. Exp. Med. 197(3): 315-322 (2003) | | | | | |

Recombinant therapeutic polypeptide drugs are usually expensive and not all countries can afford costly therapies based on such drugs. Increasing the in vivo recovery of such drugs could make the use of these products cheaper and subsequently more patients would benefit from it. Also in the case of the fusion proteins of the present invention an increased in vivo recovery would be a desirable advantage. "In vivo recovery" in the sense of the invention means the amount of product found in blood or plasma shortly after application of the product. Therefore, for detection of the in vivo recovery in general the plasma content is determined few minutes (e.g. 5 or 15 min) after application of the product.
Although it is desirable to have a high in vivo recovery and a long half-life for a non-activated coagulation factor, it is of advantage to limit the half-life of a coagulation factor after its activation respectively the activation of its co-factor in order to avoid a prothrombotic risk. Therefore, after the coagulation process has been initiated the half-life of the active, respectively activated, therapeutic fusion protein should again be reduced. This can either be achieved by inactivation or elimination of the factor.

Inactivation according to the present invention means the decrease of activity of the therapeutic polypeptide e.g. caused by reaction respectively complex formation of the therapeutic polypeptide, a coagulation factor for instance, and an inhibitor of the corresponding coagulation factor or inactivation by further proteolytic cleavage as e.g. known in the case of FVIII and FV.
The inactivation rate of an activated therapeutic fusion protein is defined as the rate the activity is declining e.g. by reaction with inhibitors or by proteolytic inactivation. The inactivation rate may be measured by following the molar specific activity of the coagulation factor over time in the presence of physiologic amounts of inhibitors of this coagulation factor. Alternatively, the inactivation rate may be determined after application of the activated product to an animal followed by testing of plasma samples at an appropriate time frame using activity and antigen assays.

The elimination rate of an activated therapeutic fusion protein is defined as the rate the polypeptide is eliminated from the circulation of humans or animals. The elimination rate may be determined by measuring the pharmacokinetics of the active, respectively activated, therapeutic fusion protein after intravenous application. Using an antigen assay, the elimination by direct removal from the circulation can be determined, using an activity assay in addition, a specific removal and inactivation rate may be determined.

According to this invention the therapeutic polypeptide moiety is coupled to the HLEP moiety by a cleavable peptidic linker. The linker should be non-immunogenic and should be flexible enough to allow cleavage by proteases. The cleavage of the linker should proceed comparably fast as the activation of the therapeutic polypeptide within the fusion protein, should this be a zymogen.

The cleavable linker preferably comprises a sequence derived from
a) the therapeutic polypeptide to be applied itself should this contain proteolytic cleavage sites e.g. used for activation of the therapeutic polypeptide,
b) a substrate polypeptide of this therapeutic polypeptide or
c) a substrate polypeptide cleaved by a protease which is activated or formed by the direct or indirect involvement of the therapeutic polypeptide.

The linker region in a more preferred embodiment comprises a sequence of the therapeutic polypeptide to be applied, which should result in a decreased risk of neoantigenicity formation of the construct and in a comparable kinetics of linker cleavage and activation of the therapeutic polypeptide should this be a zymogen.

In a preferred embodiment, the therapeutic polypeptide is FIX zymogen and the HLEP is albumin. In this case the linker sequence is either derived from the sequences of the activation regions of FIX, from the cleavage region of any substrate of FIX like FX or from the cleavage region of any substrate polypeptide that is cleaved by a protease in whose activation FIXa is involved.

In a highly preferred embodiment the linker peptide is derived from FIX itself. In another preferred embodiment the linker sequence comprises two cleavage sequences that can be cleaved by FXIa or FVIIa/TF, two physiologically relevant activators of FIX.

Exemplary combinations of therapeutic polypeptide, cleavable linker and HLEP include the constructs listed in tables 3a and 3b but are not limited to these:

**Table 3a: Examples of possible constructs**

| **Coagulation factor** | **Linker** | **HLEP** | **Linker derived from (with modifications, if applicable)** | **SEQ ID NO:** |
|---|---|---|---|---|
| | **Linker not or not sufficiently fast cleavable** | | | |
| FIX | - | HSA | | |
| FIX | RI | HSA | | |
| FIX | (GGS)nGS | HSA | | |
| FIX | SS(GGS)₇ GS | HSA | | 30 |
| FIX | SSNGS(GGS)₃ NGS(GGS)₃ GGNGS | HSA | | 31 |
| | | | | |

| | **Linker with one cleavage site** | | | |
|---|---|---|---|---|
| FIX | **R** AETVFPDVDYVNSTEA | HSA | FIX | 32 |
| FIX | **R** AETVFPDVDYVNS | HSA | FIX | 33 |
| FIX | **R** AEAVFPDVDYVNSTEA | HSA | FIX | 34 |
| FIX | **R** AEAVFPDVDYVNSTEA GS | HSA | FIX | 35 |
| FIX (1-412) | SVSQTSKLT**R** AETVFPDVD | HSA | FIX | 36 |
| FIX (1-412) | SVSQTSKLT**R** AETVFPDVD GS | HSA | FIX | 37 |
| FIX | SVSQTSKLT**R** AETVFPDVD | HSA | FIX | 38 |
| FIX | SVSQTSKLT**R** AETVFPDVD GS | HSA | FIX | 39 |
| FIX | SVSQTSKLT**R** AETVFPDVD NGS | HSA | FIX | 40 |
| FIX | SVSQTSKLT**R** GETVFPDVD | HSA | FIX | 41 |
| FIX | SVSQTSKLT**R** TETVFPDVD | HSA | FIX | 42 |
| FIX | SVSQTSKLT**R** SETVFPDVD | HSA | FIX | 43 |
| FIX | SVSQTSKLT**R** LETVFPDVD | HSA | FIX | 44 |
| FIX | SVSQTSKLT**R** TEAVFPDVD | HSA | FIX | 45 |
| FIX | SVSQTSKLT**R** GEAVFPDVD | HSA | FIX | 46 |
| FIX | SKLT**R** AETVFPDVD | HSA | FIX | 47 |
| FIX | QSFNDFT**R** WGGED | HSA | FIX | 48 |
| FIX | QSFNDFT**R** WGGED GS | HSA | FIX | 49 |
| FIX | QSFNDFT**R** TVGGED | HSA | FIX | 50 |
| FIX | QSFNDFT**R** LVGGED | HSA | FIX | 51 |
| FIX | QSFNDFT**R** GVGGED | HSA | FIX | 52 |
| FIX | QSFNDFT**R** WGGED NGS | HSA | FIX | 53 |
| FIX | QSFNDFT**R** WGGEDN | HSA | FIX | 54 |
| FIX | **R** IVGGQE | HSA | FX | 55 |
| FIX | **R** LVGGQE | HSA | FX | 56 |
| FIX | **R** TVGGQE | HSA | FX | 57 |
| FIX | **R** WGGQE | HSA | FX | 58 |
| FIX | **R**AVGGQE | HSA | FX | 59 |
| FIX | **R** GVGGQE | HSA | FX | 60 |
| FIX | PERGDNNLT**R** IVGGQE | HSA | FX | 61 |
| FIX | SVSQTSKLT**R** AETVFPDVD | Fc | FIX | 62 |
| FIX | QSFNDFT**R** WGGED N | Fc | FIX | 63 |
| FIX(1-412) | SVSQTSKLT**R** AETVFPDVD | Fc | FIX | 64 |
| FIX | KRNASKPQG**R** IVGGKV | HSA | FVII | 65 |
| FIX | PEEPQL**R** MKNNEEAED | HSA | FVIII | 66 |
| FIX | DNSPSFIQI**R** SVAKKHPKT | HSA | FVIII | 67 |
| FIX | LSKNNAIEP**R** SFSQNSRHPS | HSA | FVIII | 68 |
| FIX | DEDENQSP**R** SFQKKTRHYFIA | HSA | FVIII | 69 |
| FIX | SPHVLRN**R** AQSGSVPQ | HSA | FVIII | 70 |
| FVII or FVIIa | PEEPQL**R** MKNNEEAEDYDDDLTDS | HSA | FVIII | 71 |
| FVII or FVIIa | DDDNSPSFIQI**R** SVAKKHPKTWVHYAAEEED | HSA | FVIII | 72 |
| FVII or FVIIa | LSKNNAIEP**R** SFSQNSRHPSTRQKQFNA | HSA | FVIII | 73 |
| FVII or FVIIa | DEDENQSP**R** SFQKKTRHYFIAA | HSA | FVIII | 74 |
| FVII or FVIIa | DYGMSSSPHVLRN**R** AQSGSVPQFKKWFQEFT | HSA | FVIII | 75 |
| FVIII | Derived from cleavage sites of FVIII, FIX, or Fibrinogen | HSA | FVIII, FIX or Fgn | |
| VWF | Derived from cleavage sites of VWF, FVIII, or FIX | HSA | FIX, FVIII, VWF | |
| VWF | DIYDEDENQSPR SFQKKTRHYFIA | HSA | FVIII | 76 |
| VWF | DNSPSFIQIR SVAKKHP | HSA | FVIII | 77 |
| VWF | LSKNNAIEPR SFSQNSRHPS | HSA | FVIII | 78 |

**Table 3b: Examples of possible constructs with two or more cleavage sites**

| **Coagulation factor** | **Linker** | **HLEP** | **Linker derived from (partially incl. Modifications)** | **SEQ ID NO:** |
|---|---|---|---|---|
| | **Linker with two cleavage sites** | | | |
| FIX | SVSQTSKLT**R** AETVFPDV TQPERGDNNLT**R** IVGGQE | HSA | FIX, FX | 79 |
| FIX | SKLT**R** AETVFPDNNLT**R** IVGGQE | HSA | FIX, FX | 80 |
| FIX | **R** AETVFPDV TQPERGDNNLT**R** IVGGQE | HSA | FIX, FX | 81 |
| FIX | **R** AETVFPERGDNNLT**R** IVGGQE | HSA | FIX, FX | 82 |
| FIX | SVSQTSKLT**R** AETVFPDVDYV NNLT**R** IVGGQE | HSA | FIX, FX | 83 |
| FIX | SVSQTSKLT**R** AETVFPDVD NNLT**R** IVGGQE | HSA | FIX, FX | 84 |
| FIX | SVSQTSKLT**R** AETVFPDVD NNLT**R** IVGGQE | HSA | FIX, FX | 85 |
| FIX | SVSQTSKLTRAETVFPDVDYVNSTEAETILDNITQSTQSFND FT**R**WGGEDA | HSA | FIX | 86 |
| FIX | SVSQTSKLT**R** AETVFPDV QSFNDFT**R** WGGED | HSA | FIX | 87 |
| FIX | SVSQTSKLT**R** AETVFPDVD SFNDFT**R** WGGED | HSA | FIX | 88 |
| FIX | SVSQTSKLT**R** AETVFPDV NASKPQG**R** IVGGKV | HSA | FIX and FVII | 89 |
| FIX | SVSQTSKLT**R** AETVFPDV NASKPQG**R** LVGGKV | HSA | FIX and FVII | 90 |
| FIX | SVSQTSKLT**R** AETVFPDV NASKPOG**R** TVGGKV | HSA | FIX and FVII | 91 |
| FIX | SVSQTSKLT**R** AETVFPDVD | Fc | | 92 |

Variants and fragments of the described linkers are also encompassed in the present invention as long as the linker can still be cleaved by the protease or the proteases, which cleave the linkers of tables 3a and 3b or by the type of proteases defined above. The term "variants" includes insertions, deletions and substitutions, either conservative or non-conservative.

Other combinations of the cleavage sequences described above and their variants shall be included in the present invention.

In another embodiment, amino acid substitutions are included that change the posttranslational modification pattern of the peptide linker. These e.g. can be substitutions of amino acids that are glycosylated, sulphated, or phosphorylated.

In another embodiment of the invention the peptidic linker between the therapeutic polypeptide and the HLEP moiety contains consensus sites for the addition of posttranslational modifications. Preferably such modifications consist of glycosylation sites. More preferably, such modifications consist of at least one N-glycosylation site of the structure Asn - X - Ser/Thr, wherein X denotes any amino acid except proline. Even more preferably such N-glycosylation sites are inserted close to the amino and/or carboxy terminus of the peptidic linker such that they are capable to shield potential neoepitopes which might develop at the sequences where the therapeutic polypeptide moiety is transitioning into the peptidic linker and where the peptidic linker is transitioning into the albumin moiety sequence, respectively.

### Examples:

### Example 1: Generation of cDNAs encoding FIX and FIX - albumin fusion proteins

Factor IX coding sequence was amplified by PCR from a human liver cDNA library (ProQuest, Invitrogen) using primers We1403 and We1404 (SEQ ID NO 5 and 6). After a second round of PCR using primers We1405 and We1406 (SEQ ID NO 7 and 8) the resulting fragment was cloned into pCR4TOPO (Invitrogen). From there the FIX cDNA was transferred as an EcoRI Fragment into the EcoRI site of pIRESpuro3 (BD Biosciences) wherein an internal Xhol site had been deleted previously. The resulting plasmid was designated pFIX-496 and was the expression vector for factor IX wild-type.

For the generation of albumin fusion constructs the FIX cDNA was reamplified by PCR under standard conditions using primers We2610 and We2611 (SEQ ID NO 9 and 10) deleting the stop codon and introducing an Xhol site instead. The resulting FIX fragment was digested with restriction endonucleases EcoRI and Xhol and ligated into an EcoRI / BamH1 digested pIRESpuro3 together with one Xhol / BamH1 digested linker fragment as described below.
Two different glycine / serine linker fragments without internal cleavage site were generated: Oligonucleotides We2148 and We2150 (SEQ ID NO 11 and 12) were annealed in equimolar concentrations (10 pmol) under standard PCR conditions, filled up and amplified using a PCR protocol of a 2 min. initial denaturation at 94°C followed by 7 cycles of 15 sec. of denaturation at 94°C, 15 sec. of annealing at 55°C and 15 sec. of elongation at 72°C, and finalized by an extension step of 5 min at 72°C. The same procedure was performed using oligonucleotides We2156 and We2157 (SEQ ID NO 13 and 14). The resulting linker fragments were digested with restriction endonucleases Xhol and BamH1 and used separately in the above described ligation reaction. The resulting plasmids therefore contained the coding sequence for FIX and a C-terminal extension of a glycine / serine linker.
Two different cleavable linker fragments derived from the activation sites of FIX were generated: Oligonucleotides We2335 and We2336 (SEQ ID NO 15 and 16), containing the activation cleavage site of the FIX light chain / activation peptide border region, were annealed, filled and amplified as described above. The resulting linker fragment was digested with restriction endonucleases Xhol and BamH1 and used in the above described ligation reaction. The resulting plasmid therefore contained the coding sequence for FIX and a C-terminal extension of a cleavable FIX sequence (amino acids 136 to 154 of SEQ ID NO 2). In a subsequent site directed mutagenesis reaction with a commercially available mutagenesis kit (QuickChange XL Site Directed Mutagenesis Kit, Stratagene) using oligonucleotides We2636 and We2637 (SEQ ID NO 17 and 18) the Xhol site was deleted.
For generation of the second cleavable linker fragment derived from FIX, the same procedure was performed using oligonucleotides We2337 and We2338 (SEQ ID NO 19 and 20) for linker construction. The resulting linker fragment was digested with restriction endonucleases Xhol and BamH1 and used in the above described ligation reaction. The resulting plasmid now contained the coding sequence for FIX and a C-terminal extension of a cleavable FIX sequence derived from the activation cleavage site of the FIX activation peptide / heavy chain border region (amino acids 173 to 186 of SEQ ID NO 2). Oligonucleotides We2638 and We 2639 (SEQ ID NO 21 and 22) were used for deletion of the Xhol site as described above.
In the next cloning step the above generated plasmids were digested with BamH1 and a BamH1 fragment containing the cDNA of mature human albumin was inserted. This fragment had been generated by PCR on an albumin cDNA sequence using primers We1862 and We1902 (SEQ ID NO 23 and 24) under standard conditions.
The final plasmids with non-cleavable glycine/serine linkers were designated pFIX-980 (SEQ ID NO 30) and pFIX-986 (SEQ ID NO 31), respectively. The final plasmids with cleavable linkers derived from FIX sequences were designated pFIX-1088 (SEQ ID NO 40) and pFIX-1089 (SEQ ID NO 49), respectively. Their linker sequences and the C-terminal FIX and N-terminal albumin sequences are outlined below. Cleavage sites within the linkers are indicated with arrows, the FIX derived linker sequences are underlined.

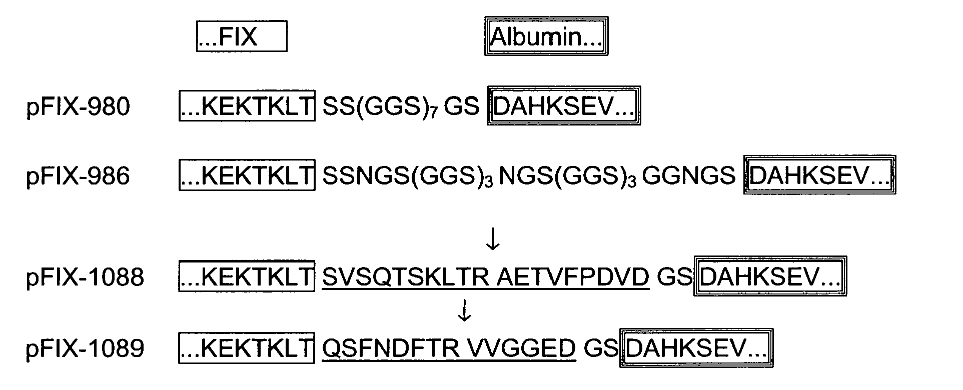

For efficient processing of the propeptide in cells expressing FIX in high amounts coexpression of furin is required (Wasley LC et al. 1993. PACE/Furin can process the vitamin K-dependent pro-factor IX precursor within the secretory pathway. J. Biol. Chem. 268:8458-8465). Furin was amplified from a liver cDNA library (Ambion) using primers We1791 and We1792 (SEQ ID NO 25 and 26). A second round of PCR using primers We1808 and We1809 (SEQ ID NO 27 and 28) yielded a furin fragment where the carboxyterminal transmembrane domain (TM) was deleted and a stop codon introduced; this fragment was cloned into pCR4TOPO (Invitrogen). From there the furinΔTM cDNA was transferred as an EcoRI/NotI Fragment into the EcoRI/NotI sites of pIRESpuro3 (BD Biosciences) wherein an internal Xhol site had been deleted previously. The resulting plasmid was designated pFu-797. This plasmid was cotransfected with all FIX constructs in a 1:5 (pFu-797 : pFIX-xxx) molar ratio. The amino acid sequence of the secreted furin encoded by pFu-797 is given as SEQ-ID NO 29.

### Example 2: Transfection and expression of FIX and FIX-albumin fusion proteins

Plasmids were grown up in E.coli TOP10 (Invitrogen) and purified using standard protocols (Qiagen). HEK-293 cells were transfected using the Lipofectamine 2000 reagent (Invitrogen) and grown up in serum-free medium (Invitrogen 293 Express) in the presence of 50 ng/ml Vitamin K and 4 µg/ml Puromycin. Transfected cell populations were spread through T-flasks into roller bottles or small scale fermenters from which supernatants were harvested for purification.

### Example 3: Purification of FIX and FIX - albumin fusion proteins

Cell culture harvest containing FIX or FIX albumin fusion protein was applied on a Q-sepharose FF column previously equilibrated with 50 mM TrisxHCl / 100 mM NaCl buffer pH 8.0. Subsequently, the column was washed with equilibration buffer containing 200 mM NaCl. Elution of the bound FIX or FIX fusion protein was achieved by a salt gradient in equilibration buffer containing 200 mM NaCl. The eluate was further purified by column chromatography on a hydroxylapatite resin. For this purpose, the eluate of the Q-Sepharose FF column was loaded on a hydroxylapatite chromatography column equilibrated with 50 mM TrisxHCl / 100 mM NaCl buffer pH 7.2. The column was washed with the same buffer and FIX or FIX-HSA were eluted using a potassium phosphate gradient at pH 7.2. The eluate was dialyzed to reduce the salt concentration and used for biochemical analysis as well as for determination of the pharmacokinetic parameters. FIX antigen and activity were determined as described in example 4.

### Example 4: Determination of FIX antigen and activity.

FIX activity was determined as clotting activity using commercially available aPTT reagents (Pathromtin SL and FIX depleted plasma, Dade Behring).
FIX antigen was determined by an ELISA acc. to standard protocols known to those skilled in the art. Briefly, microplates were incubated with 100 µL per well of the capture antibody (Paired antibodies for FIX ELISA 1:200, Cedarlane) overnight at ambient temperature. After washing plates three times with washing buffer B (Sigma P3563), each well was incubated with 200 µL blocking buffer C (Sigma P3688) for one hour at ambient temperature. After another three wash steps with buffer B, serial dilutions of the test sample in buffer B as well as serial dilutions of a substandard (SHP) in buffer B (volumes per well: 100 µL) were incubated for two hours at ambient temperature. After three wash steps with buffer B, 100 µL of a 1:200 dilution of the detection antibody (Paired antibodies for FIX ELISA, peroxidase labelled, Cedarlane) in buffer B were added to each well and incubated for another two hours at ambient temperature. After three wash steps with buffer B, 100 µL of substrate solution (TMB, Dade Behring, OUVF) were added per well and incubated for 30 minutes at ambient temperature in the dark. Addition of 100 µL undiluted stop solution (Dade Behring, OSFA) prepared the samples for reading in a suitable microplate reader at 450 nm wavelength. Concentrations of test samples were then calculated using the standard curve with standard human plasma as reference.

### Example 5: Comparison of FIX and FIX - albumin fusion proteins in respect to molar specific activity, in vivo half-life and in vivo recovery in rats or rabbits

Purified recombinant wild type FIX (rFIX 496/797) and FIX-albumin fusion proteins (rFIX 980/797, rFIX 986/797, rFlX- 1088/797 and rFIX 1089/797) were tested for FIX activity in a clotting assay as described above. In parallel the difference of the optical density at 280 and 320 nm was determined as a measure for protein concentration (OD280-320). The ratios of activity per OD280-320 were calculated and based on the molar optical densities the molar specific activities were calculated. In the following table the results are summarized.

**Table 4: Molar specific activities of wt FIX compared to FIX-albumin fusions**

| | Linker | Optical density (OD280-320) | FIX clotting activity (IU/mL) | Activity/OD (IU/mL/OD) | **Molar specific activity* (IU/nmol)** |
|---|---|---|---|---|---|
| rFIX, wt | - | 0,3798 | 21,2 | 55,8 | **4,23** |
| rFIX-HSA (non-cleavable, 980/797) | (GGS)ₙ | 1,1122 | 3,4 | 3,0 | **0,35** |
| rFIX-HSA (non-cleavable, 986/797) | (GGS)ₙ + Glyc-sites | 0,8107 | 3,2 | 4,0 | **0,45** |
| rFIX-HSA (cleavable, 1088/797) | FXIa cleavable | 0,3421 | 11,9 | 34,8 | **3,95** |
| rFIX-HSA (cleavable, 1089/797) | FXIa cleavable | 0,4512 | 11,3 | 25,0 | **2,84** |

| | | | | | |
|---|---|---|---|---|---|
| * Molar specific activity based on activity, optical density and the following molar optical densities: Molar optical density of FIX: OD(280nm, 1 mol/L) = 75 810 Molar optical density of albumin: OD(280nm, 1 mol/L) = 37 791 Molar optical density of FIX-albumin fusion protein: OD(280nm, 1 mol/L) = 113 601 | | | | | |

Taking the results summarized in Table 4 into account, it is surprising that two constructs that were generated according to the present invention show highly increased molar specific activities compared to the fusion proteins with non-cleavable linkers and a nearly unchanged molar specific activity compared to wild type rFIX.

In addition to determination of molar specific activity, the polypeptides described above were administered intravenously to narcotized CD / Lewis rats (6 rats per substance) and/or rabbits (4 rabbits per substance) with a dose of 50 IU/kg body weight. Blood samples were drawn prior to test substance application and at appropriate intervals starting at 5 minutes after application of the test substances.

FIX antigen content was subsequently quantified by an ELISA assay specific for human Factor IX (see above). The mean values of the respective groups were used to calculate in vivo recovery after 5 min. Half-lives for each protein were calculated using the time points of the beta phase of elimination according to the formula t_{1/2} = In2 / k, whereas k is the slope of the regression line.

Calculated in vivo half-lives are summarized in table 5. In rats as well as in rabbits the in vivo half-lives of the F IX fusion proteins were found to be significantly increased in comparison to the non-fused recombinant F IX prepared in house or in comparison to the commercially available recombinant FIX product BeneFIX^{®}. The in vivo half-lives of the fusion proteins compared to BeneFiX^{®} were increased to about 200-400%, depending on the animal species or construct used (Table 5).

To evaluate the in vivo recovery, the FIX antigen levels measured per mL of plasma at their maximum concentrations after intravenous application (t = 5 min) were related to the amount of product applied per kg. Alternatively, a percentage was calculated by relating the determined antigen level (IU/mL) post infusion to the theoretical product level expected at 100 % recovery (product applied per kg divided by an assumed plasma volume of 40 mL per kg). The in vivo recoveries (IVR) of the FIX-albumin fusion proteins were significantly higher than the IVR of rFIX (496/797) or BeneFIX^{®} (Table 6).

**Table 5:**

| Terminal in vivo half-lifes of FIX preparations derived from recombinant expression (BeneFIX^{®}, rFIX 496/797) and FIX albumin fusion proteins (rFIX 980/797, rFIX 986/797, rFIX 1088/797, and rFIX 1089/797) after intravenous application of 50 IU/kg into rats and/or 50 IU/kg into rabbits, respectively. | | | | |
|---|---|---|---|---|
| | **Rat experiments** PSR18-05, PSR06-05, PSR02-05 | | **Rabbit experiment** PSK11-05 | |
| | Terminal half-life (h) | relative to BeneFIX [%] | Terminal half-life (h) | relative to BeneFIX [%] |
| rFIX 496/797 | 4,5* | 91 | n.t. | n.t. |
| rFIX 980/797 | 11,6* | 234 | 36,9° | 410 |
| rFIX 986/797 | 10,5* | 212 | n.t. | n.t. |
| rFIX 1088/797 | 8,3* | 168 | n.t. | n.t. |
| rFIX 1089/797 | 10,5* | 212 | n.t. | n.t. |
| BeneFIX | 4,95* (mean of 5,3 and 4,6) | 100 | 9,0° | 100 |

| | | | | |
|---|---|---|---|---|
| * Determined between 120 and 1440 min ° Determined between 4 and 96 h | | | | |

**Table 6:**

| In vivo recoveries (amount of substance 5 minutes post application) of recombinant FIX preparations (BeneFIX, rFIX 496/797) and FIX albumin fusion proteins (rFIX 1088/797, rFIX 1089/797) after intravenous application of 50 IU /kg into rats. The percentage of in vivo recovery also was calculated based on an assumed plasma volume of 40 mL/kg. | | |
|---|---|---|
| | rat experiment | |
| | in vivo recovery IU/dL per IU/kg / [%]* | relative to BeneFIX [%] |
| rFIX 496/797 | 0.462 / 18.5 | 74.6 |
| rFIX 1088/797 | 1.034 / 41.4 | 166.5 |
| rFIX 1089/797 | 1.063/42.5 | 171.2 |
| BeneFIX | 0.621 / 24.8 | 100 |
| | | |

| | | |
|---|---|---|
| * Calculated based on a plasma volume of 40 mL/kg | | |

## Claims

1. Therapeutic fusion protein comprising
a) a coagulation factor, its variants or derivatives,
b) a half-life enhancing polypeptide selected from the group consisting of albumin including variants and derivatives thereof and immunoglobulins without antigen binding domain including variants and derivatives thereof and
c) a peptidic linker which joins the coagulation factor and the half-life enhancing polypeptide;
wherein the peptidic linker is cleavable by proteases involved in coagulation or activated by coagulation enzymes and in that the therapeutic fusion protein has in comparison to the respective therapeutic fusion protein without a cleavable linker
i) an increased molar specific activity of the coagulation factor and/or
ii) an increased inactivation rate of the coagulation factor after its activation respectively cleavage and/or
iii) an increased elimination rate of the coagulation factor after proteolytical cleavage of the therapeutic fusion protein.

2. Therapeutic fusion protein according to claim 1 wherein said fusion protein has a higher in vivo recovery compared to the in vivo recovery of the respective coagulation factor not being fused to a second protein.

3. Therapeutic fusion protein according to claims 1 and 2 wherein the coagulation factor is a vitamin-K dependent coagulation factor

4. Therapeutic fusion protein according to claims 1 to 3 wherein the coagulation factor is FVIIa or FIX.

5. Therapeutic fusion protein according to claims 1 to 4 wherein the half-life enhancing polypeptide is an immunoglobulin or a fragment or derivative thereof without an antigen binding domain.

6. Therapeutic fusion protein according to claims 1 to 5 wherein the linker is cleavable by FXIa and/or FVIIa/TF.

7. Therapeutic fusion protein according to claims 1 to 6, wherein the molar specific activity of the therapeutic fusion protein is at least 25% increased compared to that of the therapeutic fusion protein without a cleavable linker.

8. Therapeutic fusion protein according to claims 1 to 7, wherein the inactivation rate of the coagulation factor after cleavage of the peptidic linker which links the coagulation factor to the half-life enhancing polypeptide is increased by at least 10% as compared to the inactivation rate of the coagulation factor in a corresponding therapeutic fusion protein without a cleavable linker.

9. Therapeutic fusion protein according to claims 1 to 8, wherein the elimination rate of the coagulation factor after cleavage of the peptidic linker which links the coagulation factor to the half-life enhancing polypeptide is increased by at least 10% as compared to the elimination rate of the coagulation factor in a corresponding therapeutic fusion protein without a cleavable linker.

10. Therapeutic fusion protein according to claims 1 to 9, wherein the linker is cleavable by the protease(s), which activate(s) the coagulation factor.

11. Therapeutic fusion protein according to claim 10, wherein the kinetics of the linker cleavage by the protease(s) is not delayed by more than factor 3 compared to the kinetics of the activation of said coagulation factor.

12. Therapeutic fusion protein according to claims 1 to 11, wherein the linker is cleavable by the protease(s), that are activated upon involvement of the coagulation factor.

13. Therapeutic fusion protein according to claims 1 to 12, wherein the linker is cleavable by FXIa and/or by FVIIa/TF and the coagulation factor is FIX.

14. Therapeutic fusion protein according to claims 1 to 13, wherein the linker is cleavable by FXa and/or by FVIIa/TF and the coagulation factor is FVIIa.

15. Therapeutic fusion protein(s) according to any of the previous claims, wherein the linker comprises a sequence selected from the group of tables 3a and 3b

16. Therapeutic fusion protein(s) according to any of the previous claims for use as a pharmaceutical substance.

17. A polynucleotide encoding a therapeutic fusion protein according to any one of claims 1 to 15.

18. A plasmid or vector comprising a nucleic acid according to claim 17.

19. A plasmid or vector according to claim 18, which is an expression vector.

20. A plasmid or vector according to claim 18, which is a transfer vector for use in human gene therapy.

21. A host cell comprising a polynucleotide according to claim 17 or a plasmid or vector according to any one of claims 18 to 20.

22. A method of producing a therapeutic fusion protein according to any one of claims 1 to 15, comprising culturing host cells according to claim 21 under conditions such that the therapeutic fusion protein is expressed, and optionally recovering the therapeutic fusion protein from the host cells or from the culture medium.

23. A pharmaceutical composition comprising a therapeutic fusion protein according to any one of claims 1 to 15, a polynucleotide according to claim 17, or a plasmid or vector according to any one of claims 18 to 20.

24. The use of a therapeutic fusion protein according to any one of claims 1 to 15, of a polynucleotide according to claim 17, of a plasmid or vector according to any one of claims 18 to 20, or of a host cell according to claim 21 for the manufacture of a medicament for the treatment or prevention of a blood coagulation disorder.

25. The use according to claim 24, wherein the blood coagulation disorder is hemophilia B.

26. The use according to claim 24, wherein the blood coagulation disorder is FVII and/or FVIIa deficiency.

27. The use according to claim 24, wherein the blood coagulation disorder is hemophilia A.

28. The use according to any one of claims 24 to 27, wherein the treatment comprises human gene therapy.

29. The use of a therapeutic fusion protein according to any one of claims 1 to 15, of a polynucleotide according to claim 17, of a plasmid or vector according to any one of claims 18 to 20, or of a host cell according to claim 21 for the manufacturing of a medicament with procoagulant properties.
